# EUROPÄISCHE PATENTSCHRIFT

(11) **EP 0 952 853 B1**
(45) Veröffentlichungstag und Bekanntmachung des Hinweises auf die Patenterteilung: **19.03.2003**
(21) Anmeldenummer: 96946222.5
(22) Anmeldetag: 20.12.1996
(51) Int. Cl.: A61K 47/48, A61P 35/00

(54) **KONJUGAT, UMFASSEND EINEN WIRKSTOFF, EIN POLYPEPTID UND EINEN POLYETHER**
CONJUGATE COMPRISING AN ACTIVE AGENT, A POLYPEPTIDE AND A POLYETHER
CONJUGUE COMPRENANT UN PRINCIPE ACTIF, UN POLYPEPTIDE ET UN POLYETHER

(30) Priorität: 21.12.1995 DE 19548114
(43) Veröffentlichungstag der Anmeldung: 03.11.1999
(73) Patentinhaber: Deutsches Krebsforschungszentrum Stiftung des öffentlichen Rechts, 69120 Heidelberg (DE)
(72) Erfinder: SINN, Hannsjörg, D-69118 Wiesloch (DE); MAIER-BORST, Wolfgang, D-69221 Dossenheim (DE); SCHRENK, Hans-Hermann, D-67728 Zeiskamm (DE); STEHLE, Gerd, D-89584 Ehingen (DE); FIEBIG, Heinz-H., D-79110 Freiburg (DE)
(74) Vertreter: Schüssler, Andrea, Dr.
(86) Internationale Anmeldenummer: DE9602487
(87) Internationale Veröffentlichungsnummer: WO97023240

(56) Entgegenhaltungen:
- EP-A- 0 397 307
- WO-A-94/05203
- WO-A-94/28940
- WO-A-96/00079
- WO-A-96/00088
- WO-A-96/00588
- DE-A- 4 017 439
- ADV. DRUG DELIVERY REV. (1995), 16(2,3), 335-48 CODEN: ADDREP;ISSN: 0169-409X, 1995, XP002037424 BOGDANOV, ALEXEI A. JR. ET AL: "Long-circulating blood pool imaging agents"
- BIOCONJUGATE CHEM. (1996), 7(1), 144-9 CODEN: BCCHES;ISSN: 1043-1802, 1996, XP002037425 BOGDANOV, A. A., JR. ET AL: "An Adduct of cis-Diaminodichloroplatinum(II) and Polyethylene glycol-poly(L-lysine)-Succinate: Synthesis and Cytotoxic Properties"
- MAKROMOL. CHEM., RAPID COMMUN. (1981), 2(9-10), 637-43 CODEN: MCRCD4;ISSN: 0173-2803, 1981, XP002037426 ANZINGER, HERMANN ET AL: "Peptides with unusual side chains. Synthesis and conformation of poly(ethylene glycol)-polylysine blocks with mesogenic side groups"
- BIOCONJUGATE CHEMISTRY, Bd. 3, Nr. 4, 1.Juli 1992, Seiten 295-301, XP000288471 MASAYUKI YOKOYAMA ET AL: "PREPARATION OF MICELLE-FORMING POLYMER-DRUG CONJUGATES"
- AMERIC. J. ROENTGENOLOGY, Bd. 162, Nr. 5, - 1994 Seiten 1041-1046, XP002037427 FRANK H. ET AL. : "DETECTION OF PULMONARY EMBOLI BY USING MR ANGIOGRAPHY WITH MPEG-PL-GDDTPA: AN EXPERIMENTAL STUDY IN RABBITS."
- ADVANCED DRUG DELIVERY REVIEWS, Bd. 16, Nr. 2-3, 1995, Seiten 157-182, XP002037428 ZALIPSKY: "CHEMISTRY OF POLYETHYLENE GLYCOL CONJUGATES."
- RADIOLOGY, Bd. 196, Nr. 1, 1995, Seiten 239-244, XP000606052 GUPTA H. ET AL.: "EXPERIMENTAL GASTROINTESTINAL HEMORRHAGE: DETECTION WITH CONTRAST-ENHANCED MR IMAGING AND SCINTIGRAPHY."
- RADIOLOGY, Bd. 197, Nr. 3, 1995, Seiten 665-669, XP002037429 GUPTA H. ET AL.: "INFLAMMATION: IIMAGING WITH METHOXY POLY(ETHYLNEN GLYCOL)-POLY-L-LYSINE-DTPA, A LONG CIRCULATING GRAFT POLYMER."

## Beschreibung

Die Erfindung betrifft Konjugate, umfassend einen Wirkstoff, ein Polypeptid und mehrere Polyether, Verfahren zur Herstellung solcher Konjugate sowie ihre Verwendung.

Es besteht ein großes Bedürfnis, Wirkstoffe an ihrem Wirkort anzureichern. Dies trifft insbesondere für Wirkstoffe zur Behandlung von Tumoren bzw. entzündeten Geweben zu. Vielfach wird versucht, die Wirkstoff-Anreicherung durch Verabreichung hoher Wirkstoff-Mengen zu erlangen. Dies führt allerdings zu erheblichen Nebenwirkungen beim Patienten, insbesondere seiner Leber und Niere.

Yokoyama et al. (Bioconjugate Chemistry 3 (1992), No. 4, S. 295-301) und EP-A-0 397 307 beschreiben micellen-bildende Polymer-Wirkstoff-Konjugate, wobei die Größe der Micellen 20 bis 200 nm im Durchmesser beträgt und erst durch Zugabe von SDS unter extremen Bedingungen wieder aufgelöst werden kann.

Der vorliegenden Erfindung liegt somit die Aufgabe zugrunde, ein Mittel bereitzustellen, mit dem Wirkstoffe an ihrem Wirkort angereichert werden können.

Erfindungsgemäß wird dies durch die Gegenstände in den Patentansprüchen erreicht.

Gegenstand der vorliegenden Erfindung ist somit ein Konjugat, umfassend einen Wirkstoff, ein Polypeptid und mehrere Polyether.

Der Ausdruck "Wirkstoff" umfaßt photoaktive Substanzen, zur Fluoreszenz-fähige Substanzen, und/oder Substanzen als Radiosensitizer. Beispiele der photoaktiven Substanzen sind Porphyrine, wie o-, m- und/oder p-Tetracarboxyphenylporphyrin (TCPP), Chlorine und Bakteriochlorine. Beispiele der zur Fluoreszenz-fähigen Substanzen sind Fluoreszenzfarbstoffe, wie Fluorescein, Aminofluorescein (AFI), sowie Derivate und Analoga davon. Ein Beispiel der Radiosensitizer-Substanz ist m-Aminobenzoesäureamid.

Von vorstehenden Wirkstoffen können einer oder mehrere im erfindungsgemäßen Konjugat vorliegen. Bei mehreren können diese gleich oder verschieden voneinander sein.

Der Ausdruck "Polypeptid" umfaßt Polylysin (PL), Polytyrosin, Polyasparaginsäure und Polyglutaminsäure, die bis zu 50 Aminosäuren, vorzugsweise 7 bis 30 Aminosäuren, besonders bevorzugt 10 bis 30 Aminosäuren aufweisen.

Dem Fachmann sind vorstehende Polypeptide bekannt. Sie können käuflich erworben werden oder mit bekannten Techniken, z.B. der Merrifield-Technik, hergestellt werden.

Im erfindungsgemäßen Konjugat liegen mehrere Polyether vor. Diese schützen das Konjugat insbesondere vor der Ausscheidung über die Niere und verleihen ihm eine gute Wasserlöslichkeit. Vorzugsweise weist der Polyether ein Molekulargewicht von 2000 bis 5000 Dalton auf. Der Polyether kann an der endständigen Hydroxylgruppe mit einer C₁-C₁₂-Alkylgruppe, insbesondere Methylgruppe, verethert oder verestert sein. Der Polyether ist vorzugsweise ein Polyethylenglykol (PEG), wie Methoxypolyethylenglykol, und wird über aktive Gruppen wie Methoxypolyethylenglykol-p-nitrophenylcarbonat Methoxypolyethylenglykolsuccinimidylsuccinat, Methoxypolyethylenglykoltresylat, Methoxypolyoxyethylenamin, Methoxypolyoxyethylencarbonsäure und Methoxypolyoxyethylenimidazolcarbonyl, an das Polypeptid gebunden.

Im erfindungsgemäßen Konjugat liegen vorzugweise 2-6 vorstehender Polyether vor. Diese können gleich oder verschieden sein. Liegen mehrere Polyether vor, beträgt günstigerweise die Summe der Molekulargewichte dieser 10 000 bis 50 000 Dalton.

Im erfindungsgemäßen Konjugat können die einzelnen Komponenten direkt, z.B. kovalent über eine Säureamid- oder Säureesterbindung, oder indirekt über einen Linker, wie Cyanurchlorid, verbunden sein. Vorzugsweise ist sowohl der Wirkstoff als auch der Polyether an das Polypeptid gebunden.

Vorstehende Komponenten des erfindungsgemäßen Konjugats sind als Edukte angegeben. Im erfindungsgemäßen Konjugat liegen sie daher derivatisiert vor.

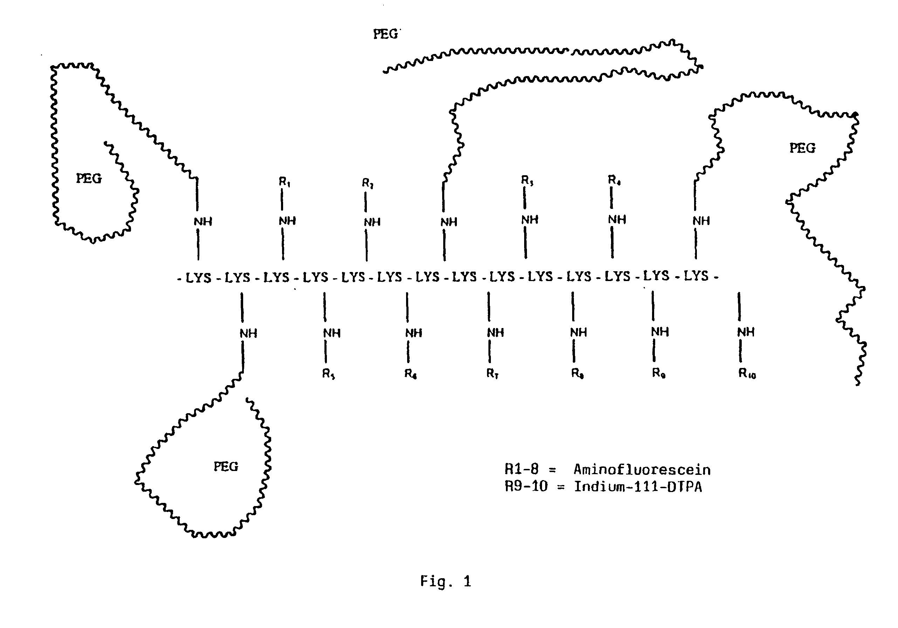

Beispiele erfindungsgemäßer Konjugate sind in Figur 1 und Beispiel 1 angegeben.

Erfindungsgemäß wird auch ein Verfahren zur Herstellung eines vorstehenden Konjugats bereitgestellt. In einem solchen werden das Polypeptid, der Wirkstoff und der Polyether in üblicher Weise, vorzugsweise kovalent, miteinander verbunden. Es wird auf die Herstellung eines erfindungsgmäßen Konjugats in Beispiel 1 verwiesen.

Erfindungsgemäße Konjugate zeichnen sich dadurch aus, daß sie sich gut in Tumoren und von endzündlichen Erkrankungen geschädigten Geweben anreichern. Ferner können sie mit den unterschiedlichsten Wirkstoffen, insbesondere gleichzeitig, beladen sein, ohne immunogen zu sein. Dies ist ein großer Vorteil gegenüber Konjugaten aus dem Stand der Technik, die oftmals, wenn mehrere Wirkstoffe gleichzeitig vorliegen, ungewünschte Immunreaktionen beim Patienten hervorrufen. Somit eignen sich erfindungsgemäße Konjugate gut zur Behandlung von Erkrankungen, insbesondere von Tumor- und entzündlichen Erkrankungen.

Desweiteren eignen sich erfindungsgemäße Konjugate gut zur Diagnose von Erkrankungen, insbesondere von Tumor- und entzündlichen Erkrankungen. Beispielsweise können in erfindungsgemäßen Konjugaten der Wirkstoff eine zur Fluoreszenz-fähige Substanz sein. Diese Konjugate werden dem Tumorpatienten beispielsweise vor der Operation verabreicht. Die erfindungsgemäßen Konjugate reichern sich dann im Tumor an. Während der Operation kann die zur Fluoreszenz-fähige Substanz mit Licht, z.B. einer UV-Lampe, zur Fluoreszenz angeregt werden. Somit kann gesundes Gewebe von Tumorgewebe unterschieden werden.

### Kurze Beschreibung der Zeichnung

- Figur 1: zeigt ein erfindungsgemäßes Konjugat, umfassend Polylysin (PL), 4 Polyethylenglykole (PEG), 8 Aminofluoresceine (AFI) und 2 mit ¹¹¹In markierte Diethylentriaminpentaacetate (DTPA), wobei AFI über Cyanurchlorid an freie Aminogruppen des PL gebunden sind, und DTPA über eine Säureamidbrücke an PL gebunden sind.
- Figur 2: zeigt die mittels Szintigraphie gemessene Verteilung des erfindungsgemäßen Konjugats von Figur 1 in Tumorgewebe.

Die folgenden Beispiele erläutern die Erfindung.

### Beispiel 1: Herstellung eines erfindungsgemäßen Konjugats, umfassend PL, 8 AFI, 2 DTPA und 4 Methoxypolyethylenglykole (MPEG)

50 mg 5([4,6-Dichlorotriazin-2-yl]-amino)-fluorescein (AFI, FG 531.7; Sigma) wurden in 4ml Dimethylsulfoxid (DMSO) gelöst und zu 100 mg Polylysin (PL-FG ∼1000 D, Sigma), gelöst in 2ml DMSO und 2ml dest. Wasser, zugegeben. Anschließend wurde 1 ml 0,34 M NaHCO₃ zugegeben, und die leicht getrübte Lösung etwa 30 min stehengelassen.

1 g Methoxypolyethylenglykol (MPEG)-Nitrophenylcarbonat wurden in 10 ml Dioxan gelöst und zu vorstehender AFI-PL Lösung zugegeben. Dazu wurden 50µl 1 N NaOH zugegeben. Nach etwa 48 h war die Umsetzung beendet. Es wurde PL-AFL-MPEG₄ erhalten. Die Abtrennung unerwünschter Begleitstoffe erfolgte über Ultrafiltration (YM 10). Die Ermittlung des Molekulargewichts erfolgte mittels GPC in üblicher Weise.

Die Umsetzung von PL-AFI-MPEG₄ mit Diethylentriaminpentaessigsäure (DTPA), das mit ¹¹¹In zum nuklearmedizinischen Einsatz oder mit Gadolinium (Gd) als tumorgängiges MR-Kontrastmittel markiert wurde, erfolgte folgendermaßen:

Diethylentriaminpentaessigsäure (DTPA) wurde unter Erwärmung auf eine Konzentration von 20 mg/ml in DMSO gelöst. Nach dem Abkühlen der klaren Lösung auf Raumtemperatur wurde die 1,5 - 2-fache molare Menge von N,N'-Dicyclohexylcarbodiimid (DCC) und die 5-fache molare Menge von N-Hydroxysuccinimid (HSI) zugegeben. Nach einer Reaktionszeit von etwa 14-15 h war die Bildung des Succinimidylesters (DTPA-SE) abgeschlossen. Dieses wurde zur nachfolgenden Kopplung an PL-AFI-MPEG₄ eingesetzt.

Dazu wurde zu vorstehender durch Ultrafiltration gereinigten Lösung von PL-AFL-MPEG₄ sehr langsam und unter ständigem Rühren die vorstehende Lösung des DTPA-SE hinzugefügt, wobei sich die zunächst klare Lösung durch überschüssiges und nicht umgesetztes DCC und noch gelöstem Di-Cyclohexylharnstoff (DCCH), die beide in wässriger Lösung unlöslich sind, eintrübte. Nach Beendigung der Zugabe von DTPA-SE wurde das Reaktionsgemisch zur vollständigen Umsetzung weitere 60 min bei Raumtemperatur gerührt. Anschließend wurden die Trübung über eine Sterilfiltereinheit (Millipore, Stericup-GV, 0,22µm Low Binding Duropore Membrane) und die niedermolekularen wasserlöslichen Komponenten (DMSO, HSI und nicht gebundenes DTPA) durch Ultrafiltration über eine Membran mit 10 kD Ausschlußgrenze (Amicon) abgetrennt. Die analytische Reinheitskontrolle erfolgte mittels HPLC. Es wurde das erfindungsgemäße Konjugat PL-AFL-MPEG₄-DTPA₂ (A) erhalten.

Zur Markierung von (A) mit ¹¹¹In wurden zu 185 MBq ¹¹¹In(5 mCi), vorgelegt als 0,1 M salzsaure Lösung von trägerarmem ¹¹¹InCl₃, 10-20µl einer 0,2 M Na-citratlösung zugegeben. Dazu wurden etwa 5 mg (A), gelöst in 1-2 ml 0,17 M NaH-CO₃, zugegeben. Danach wurde die Reaktionsmischung durch Ultrafiltration von nicht gebundenem ¹¹¹In und Citrat abgetrennt. Das Reaktionsvolumen wurde hierzu auf 2 ml aufgefüllt und in eine Einmal-Ultrafiltrationseinheit (Centricon C 30, Amicon) überführt, wobei die Abtrennung der niedermolekularen Verunreinigungen durch Zentrifugation in einem 45° Schrägwinkelrotor erfolgt. Es wurde mit ¹¹¹In-markiertes (A) erhalten.

Die Beladung von (A) mit Gd erfolgt wie die Markierung mit ¹¹¹In, wobei nur größere Substanzmengen benötigt werden.

### Beispiel 2: Aufnahme eines erfindungsgemäßen Konjugats in Tumorgewebe.

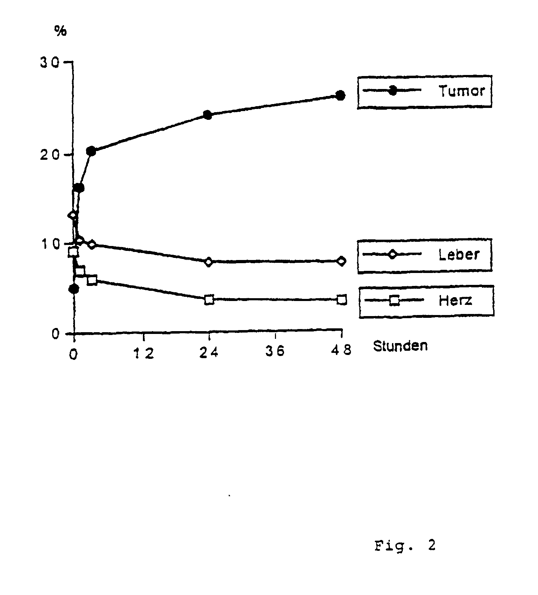

a) Etwa 70 µCi (2 mg/kg) des erfindungsgemäßen Konjugats von Fig. 1 wurden Ratten, die ein Walker-256 Karzinosarkom aufwiesen, intravenös verabreicht. Es wurde zu den Zeiten, 5 Minuten, 1, 3, 24 und 48 Stunden, szintigraphisch die Verteilung des Konjugats in den Ratten nachgewiesen. Das Ergebnis ist in Fig. 2 dargestellt.
Aus Figur 2 geht hervor, daß sich das erfindungsgemäße Konjugat im Tumor anreichert, nicht jedoch im Bereich des Herzens oder der Leber.
b) Vorstehende Ratten wurden nach 48 Stunden getötet und die Radioaktivitätsverteilung in den Organen wurde bestimmt. Die Ergebnisse sind in Tabelle 1 dargestellt.
Aus Tabelle 1 geht hervor, daß sich das erfindungsgemäße Konjugat im Tumor anreichert.

**Tabelle 1:**

| Verteilung des erfindungsgemäßen Konjugats von Fig. 1 im Blut, in bestimmten Organen und im Tumor einer Ratte. Von der im Körper zurückgebliebenen Radioaktivität befindet sich 30% im Tumor, der selbst nur 4,8% des Körpergewichts einnimmt. | | | |
|---|---|---|---|
| | Gewicht/ Volumen | Menge an Konjugat (µCi) | Anteil (%) |
| Ratte | 243,0 g | 27,5 | 100% |
| Blut | 15,4 ml | 1,32 | 4,8% |
| Tumor | 11,2 g | 8,50 | 30,9% |
| Haut | 40,2 g | 0,75 | 2,9% |
| Milz | 0,55 g | 0,23 | 0,8% |
| Nieren (zus.) | 2,38 g | 2,60 | 9,4% |
| Leber | 10,71 g | 2,82 | 10,2% |
| Magendarmtrakt | 13,30 g | 1,43 | 0,5% |

## Patentansprüche

1. Konjugat, umfassend einen Wirkstoff, ein Polypeptid und mehrere Polyether, **dadurch gekennzeichnet, daß** der Wirkstoff eine photoaktive Substanz, eine zur Fluoreszenz-fähige Substanz und/oder ein Radiosensitizer ist und das Polypeptid Polylysin, Polytyrosin, Polyasparaginsäure oder Polyglutaminsäure ist.

2. Konjugat nach Anspruch 1, **dadurch gekennzeichnet, daß** mehrere Wirkstoffe vorliegen.

3. Konjugat nach Anspruch 1 oder 2, **dadurch gekennzeichnet, daß** das Polypeptid 7 bis 30 Aminosäuren aufweist.

4. Konjugat nach einem der Ansprüche 1 bis 3, **dadurch gekennzeichnet, daß** die Polyether in der Summe ein Molekulargewicht von 10 000 bis 50 000 Dalton aufweisen.

5. Konjugat nach Anspruch 1, **dadurch gekennzeichnet, daß** 2 bis 6 Polyether vorliegen.

6. Konjugat nach Anspruch 1 bis 5, **dadurch gekennzeichnet, daß** der Polyether ein Polyethylenglykol ist.

7. Verfahren zur Herstellung eines Konjugats nach einem der Ansprüche 1 bis 6, **dadurch gekennzeichnet, daß** der Wirkstoff, das Polypeptid und die Polyether kovalent miteinander verbunden werden.

8. Verwendung eines Konjugats nach einem der Ansprüche 1 bis 6 zur Herstellung einer pharmazeutischen Zusammensetzung zur Behandlung und/oder Diagnose von Erkrankungen.

9. Verwendung nach Anspruch 8, wobei die Erkrankung eine Tumorerkrankung und/oder entzündliche Erkrankung ist.

## Claims

1. A conjugate comprising an active substance, a polypeptide and several polyethers, **characterized in that** the active substance is a photoactive substance, a substance capable of fluorescence and/or a radiosensitizer and the polypeptide is polylysine, polytyrosine, polyasparaginic acid or polyglutamic acid.

2. The conjugate according to claim 1, **characterized in that** several active substances are present.

3. The conjugate according to claim 1 or 2, **characterized in that** the polypeptide has 7 to 30 amino acids.

4. The conjugate according to any of claims 1 to 3, **characterized in that** altogether the polyethers have a molecular weight of 10,000 to 50,000 Dalton.

5. The conjugate according to claim 1, **characterized in that** 2 to 6 polyethers are present.

6. The conjugate according to claims 1 to 5, **characterized in that** the polyether is a polyethylene glycol.

7. A method of producing a conjugate according to any of claims 1 to 6, **characterized in that** the active substance, the polypeptide and the polyethers are covalently bonded with one another.

8. Use of a conjugate according to any of claims 1 to 6 for producing a pharmaceutical composition for treating and/or diagnosing diseases.

9. Use according to claim 8, wherein the disease is a tumoral disease and/or inflammatory disease.

## Revendications

1. Conjugué comprenant une substance active, un polypeptide et plusieurs polyéthers, **caractérisé en ce que** la substance active est une substance photoactive, une substance apte à la fluorescence et/ou une substance radiosensibilisante et le polypeptide est une polylysine, une polytyrosine, un poly(acide aspartique) ou un poly(acide glutamique).

2. Conjugué suivant la revendication 1, **caractérisé en ce que** plusieurs substances actives sont présentes.

3. Conjugué suivant la revendication 1 ou 2, **caractérisé en ce que** le polypeptide présente 7 à 30 aminoacides.

4. Conjugué suivant l'une des revendications 1 à 3, **caractérisé en ce que** les polyéthers totalisent un poids moléculaire de 10000 à 50000 daltons.

5. Conjugué suivant la revendication 1, **caractérisé en ce que** 2 à 6 polyéthers sont présents.

6. Conjugué suivant les revendications 1 à 5, **caractérisé en ce que** le polyéther est un polyéthylèneglycol.

7. Procédé de préparation d'un conjugué suivant l'une des revendications 1 à 6, **caractérisé en ce que** la substance active, le polypeptide et les polyéthers sont liés entre eux par covalence.

8. Utilisation d'un conjugué suivant l'une des revendications 1 à 6 pour la préparation d'une composition pharmaceutique destinée au traitement et/ou au diagnostic de maladies.

9. Utilisation suivant la revendication 8, dans laquelle la maladie est une maladie tumorale et/ou une maladie inflammatoire.
